# EUROPÄISCHE PATENTSCHRIFT

(11) **EP 1 554 390 B1**
(45) Veröffentlichungstag und Bekanntmachung des Hinweises auf die Patenterteilung: **16.07.2008**
(21) Anmeldenummer: 03775203.7
(22) Anmeldetag: 20.10.2003
(51) Int. Cl.: C12P 7/06, B01D 3/00

(54) **ANLAGE ZUR HERSTELLUNG VON ALKOHOL**
APPARATUS FOR THE PRODUCTION OF ALCOHOL
INSTALLATION DE PRODUCTION D'ALCOOL

(30) Priorität: 21.10.2002 DE 10249027
(43) Veröffentlichungstag der Anmeldung: 20.07.2005
(73) Patentinhaber: GEA Wiegand GmbH, D-76275 Ettlingen (DE)
(72) Erfinder: HOCHBERG, Ulrich, 76199 Karlsruhe (DE); SCHORMÜLLER, Matthias, 76181 Karlsruhe (DE)
(74) Vertreter: Liska, Horst
(86) Internationale Anmeldenummer: PCT/EP2003/011602
(87) Internationale Veröffentlichungsnummer: WO 2004/038031

(56) Entgegenhaltungen:
- WO-A-96/36723
- US-A- 4 287 304
- US-A- 5 545 543

## Beschreibung

Die Erfindung betrifft eine Anlage zur Herstellung von Alkohol aus Kohlehydrat oder Zucker enthaltendem Rohstoff.

Die großtechnische Herstellung von Alkohol, insbesondere Ethylalkohol, aus Kohlehydrat oder/und Zucker enthaltenden pflanzlichen Rohstoffen nach herkömmlichen Verfahren der Verflüssigung des Rohstoffs, der Vergärung zu Maische und nachfolgender Destillation des Rohalkohols ist energieaufwändig, insbesondere wenn die bei der Destillation als Rückstand anfallende Schlempe zur Herstellung von Tierfutter oder dergleichen getrocknet wird.

So sind zum Beispiel für die Erzeugung von einem Liter wasserfreiem Ethylalkohol und getrockneter Schlempe zwischen 7 und 8 kg Wasserdampf erforderlich. In Summe übersteigt die zur Alkoholherstellung erforderliche Energie bei herkömmlichen Verfahren den Energieinhalt des Alkohols und dementsprechend ist der Einsatz von Alkohol als Brennstoff in Kraftmaschinen, beispielsweise Automobilmotoren bezogen auf die Gesamtenergiebilanz unrentabel.

Die Erfindung geht aus von einer herkömmlichen Anlage zur Herstellung von Alkohol aus Körnerfrucht-Rohstoffen, welche umfasst:
- eine zumindest den die Stärke oder/und Zucker enthaltenden Kern der Körnerfrüchte zu Mehl vermahlende Mahlstation,
- eine das Mehl zu verflüssigtem Rohstoff aufschließende Verflüssigungsstation,
- eine verflüssigten Rohstoff zu Maische vergärende Fermentierungsstation,
- eine den Alkohol aus der Maische abtrennende Destillierstation und
- eine Trocknungsstation für in der Destillierstation anfallende Schlempe.

Eine solche Anlage ist aus DE 32 04 910 A bekannt. Sie umfasst eine Trockengetreide vermahlende Mühle, eine das Mehl aufschließende Verflüssigungsstation sowie eine den verflüssigten Rohstoff zu Maische vergärende Fermentierungsstation. In einer Destillierstation wird der Alkohol aus der Maische abgetrennt. Ein Dekanter trennt aus der in der Destillierstation anfallenden Schlempe einen Teil des Schlempewassers ab, welches in die Verflüssigungsstation zurückgeführt wird. Der Feststoffanteil der Schlempe wird einer Trocknungsstufe zugeführt. Der Trocknerabdampf der Trocknungsstufe wird zur Ergänzung des Wasserhaushalts der Alkoholherstellungsanlage in den Prozess rückgeführt.

Aus US 4 287 304 A ist eine weitere Alkohol-Herstellungsanlage bekannt. Sie umfasst eine Trockenvermahlungseinheit für Mais, in der Schalenanteile abgetrennt werden, eine Verflüssigungsstation für das erhaltene Maismehl, eine Fermentierungsstation, eine Destillierstation und eine Trocknungseinheit für die in der Destillierstation anfallende Schlempe. Eine ähnliche Anlage ist aus WO96/36723 A bekannt. Auch hier wird die in einer Destillationskolonne anfallende Schlempe getrocknet und zu Tierfutter weiter verarbeitet.

Schließlich ist aus DE 31 15 289 A eine Alkoholherstellungsanlage bekannt, bei welcher mittels einer Zentrifuge die vergorene und damit alkoholhaltige Maische in eine alkoholhaltige Flüssigfraktion und eine gleichfalls alkoholhaltige Feststofffraktion getrennt wird. Die Flüssigfraktion wird einer Destillationskolonne zugeführt, während die Feststofffraktion zunächst gepresst und dann in einem Trockner getrocknet wird. Der alkoholhaltige Abdampf des Trockners wird gleichfalls der Destillationskolonne zum Abtrennen des Restalkohols zugeführt. Die in der Destillationskolonne anfallende Schlempe wird in den Maischeherstellungsprozess rückgeführt. Die Destillationskolonne wird durch Frischdampf und Brüden einer auf die Destillationskolonne folgenden Verdampferstufe beheizt.

Es ist Aufgabe der Erfindung, einen Weg zu zeigen, wie Alkohol, insbesondere Ethylalkohol, und getrocknete Schlempe mit weniger Energieaufwand als bisher aus pflanzlichen Rohstoffen hergestellt werden kann.

Diese Aufgabe wird durch die in Anspruch 1 angegebenen Merkmale gelöst.

Ausgehend von der vorangegangen erläuterten Anlage zur Herstellung von Alkohol aus Körnerfrucht-Rohstoffen ist vorgesehen, dass die Mahlstation die Körnerfrüchte unter Abtrennung zumindest eines Teils ihrer den Kern umschließenden Schalenanteile zu Mehl vermahlt und die abgetrennten Schalenanteile der Trockenstation als Trägermedium für die Schlempetrocknung zugeführt sind, dass die Trocknungsstation einen die in der Destillation anfallende Schlempe endtrocknenden Trockner mit einer Taupunkttemperatur von mehr als 95°C, bevorzugt 100°C bis 105°C umfasst, und dass die Destillierstation eine mit dem Abdampf des Trockners beheizte Destillationskolonne umfasst.

Für die Erfindung sind mehrere Aspekte maßgebend, die jeweils für sich und erst recht in ihrer Kombination eine beträchtlich kostengünstigere Herstellung des Alkohols, insbesondere Ethylalkohols, aus kohlehydrathaltigen oder/und zuckerhaltigen pflanzlichen Rohstoffen wie zum Beispiel Mais, Weizen, Sorghum oder auch Melasse ermöglichen. Alkohol und getrocknete Schlempe lassen sich mit beträchtlich weniger Energie, insbesondere weniger als 50 % des bisherigen Energieaufwands herstellen.

Bei der Herstellung von Alkohol aus Körnerfrüchten, wie zum Beispiel Mais oder Weizen oder dergleichen ist eine den die Stärke oder/und den Zucker enthaltenden Kern der Körnerfrüchte unter Abtrennung zumindest eines Teils ihrer den Kern umschließenden Schalenanteile zu Mehl vermahlende Mahlstation und eine das Mehl aufschließende Verflüssigungsstation vorgesehen und die abgetrennten Schalenanteile werden der Trockenstation als Trägermedium für die Schlempetrocknung zugeführt. Wenngleich auch mit den als Kleie oder dergleichen anfallenden Schalenanteilen ein gewisser Prozenzsatz der vergärbaren Kohlehydrate oder Zuckeranteile des Rohstoffs nicht dem Gärprozess zugeführt wird, so wird dieser Umstand dadurch ausgeglichen, dass die Maische reicher an vergärbaren Kohlehydraten und an Zucker ist, so dass höherprozentiger Alkohol destilliert werden kann. Zugleich vermindert sich der Energieverbrauch in der Destillierstation.

Bei herkömmlichen, zur Schlempetrocknung eingesetzten Trocknern wird ein Teil der getrockneten Schlempe als Trägermaterial in den Trocknungsprozess zurückgeführt. Dieser Anteil kann im Rahmen der Erfindung verringert werden oder ganz entfallen, nachdem als Trägermedium die bei der Vermahlung abgetrennten Schalenanteile (Spelzen oder Kleie) dem Trockner direkt zugeführt werden. Da die abgetrennten Schalenanteile nicht der Verflüssigung zugeführt wurden, also nicht getrocknet werden müssen, verringert sich der Energieverbrauch des Trockners und es können kleinere Trockner als bisher eingesetzt werden.

Um in der Mahlstation die Schalenanteile hinreichend abtrennen zu können, wird zweckmäßigerweise eine Walzenmühle oder einer Prallstrahlmühle eingesetzt. Als Optimum zwischen Verbesserung des Trocknungsprozesses einerseits und hinreichender Alkoholausbeute andererseits hat sich ein Gewichtsverhältnis der Schalenanteile der abgetrennten Schalenanteile zum Mehl von 1:9 bis 2:8 herausgestellt. Die mittlere Korngröße der in der Mahlstation vermahlenen Körnerfrüchte sollte größer als 0,2 mm sein und liegt zweckmäßigerweise zwischen 0,5 mm bis 1 mm.

Bei der Alkoholherstellung muss die in dem pflanzlichen Rohmaterial enthaltene, zumeist gekörnte Stärke verflüssigt bzw. aufgeschlossen werden. Herkömmlich erfolgt dies unter Zusatz von Enzymen durch Dampf, mit dem das Mehl oder gegebenenfalls in einer Aufschlämmung vorliegendes Mehl über seine durch das Rohstoffmaterial bestimmte Verkleisterungstemperatur hinaus erwärmt wird. Nach einer die enzymatische Verflüssigung erlaubenden Verweilzeitspanne muss die in dem Verflüssigungsschritt entstehende "Süßmaische" wieder abgekühlt werden. Gegebenenfalls kann sich ah den Verflüssigungsschritt noch ein Vorfermentierungs- oder Verzuckerungsschritt anschließen.

Herkömmlich werden Destillationskolonnen durch Frischdampf beheizt. Die Erfindung zeigt einen Weg, wie für die Beheizung der Destillationskolonne bereits anderweitig in der Alkoholherstellungsanlage anfallende Energie genutzt werden kann. Hierzu ist vorgesehen, dass die Trocknungsstation einen die Schlempe endtrocknendan Trockner mit einer Taupunkttemperatur von mehr als 95°C, bevorzugt 100° bis 105°C umfasst und dass die Destillationsstation eine mit dem Abdampf des Trockners beheizte Destillationskolonne hat. Mit dem Abdampf eines derartigen Trockners lässt sich zumindest die den Rohalkohol abgebende Destillationskolonne ausschließlich beheizen, insbesondere wenn diese Destillationskolonne keine allzu großen Druckverluste hat, wie sich dies vorangegangen erläutert durch die CO₂-Entgasung der dieser Destillationskolonne zugeführten Maische erreichen lässt. Hohe Taupunkttemperaturen des Abgases lassen sich speziell mit einem Trockner erreichen, welcher im Wesentlichen luftfreien Abdampf erzeugt. Ein solcher Trockner kann beispielsweise als mit überhitztem Dampf betriebener Heißdampftrockner ausgebildet sein.

Bei herkömmlichen Verflüssigungsstationen wird die Stärke durch Dampf durch einen mit Frischdampf beschickten Dampfstrahlerhitzer in einem einzigen Schritt bis über die Verkleisterungstemperatur hinaus erwärmt. Für die Verflüssigung ist hierbei vergleichsweise viel Energie erforderlich.

Der Energiebedarf der Verflüssigungsstation lässt sich beträchtlich verringern, wenn die Verflüssigungsstation einen dem Produktstrom des zerkleinerten Rohrstoffs oder einer Aufschlämmung hiervon Dampf zumischenden, wenigstens eine Mischstufe umfassenden Mischkondensator, sowie einen dem Mischkondensator nachgeschalteten, dem Produktstrom Heißdampf zumischenden Dampfstrahlinjektor und einen dem Dampfstrahlinjektor nachgeschaltetem, wenigstens eine Entspannungsstufe umfassenden Entspannungskühler für den Produktstrom umfasst. Der Mischkondensator mischt dem Produktstrom den Entspannungsdampf des Entspannungskühlers zu, nutzt also die in dem Entspannungskühler aus dem Produktstrom rückgewonnene Energie für die Teilerwärmung des Produktstroms bei der Verflüssigung aus. In einer bevorzugten Ausgestaltung erwärmt der Mischkondensator den Produktstrom auf eine Temperatur unterhalb der Verkleisterungstemperatur des Rohstoffs, so dass der Dampfstrahlinjektor nur noch Erwärmungsenergie, zum Beispiel aus Frischdampf, für die Resterwärmung bis oberhalb der Verkleisterungstemperatur zuführen muss. Für diesen Resterwärmungsschritt ist vergleichsweise wenig Energie erforderlich.

Zumindest der Entspannungskühler ist bevorzugt mehrstufig ausgebildet, wobei der Mischkondensator dem Produktstrom zumindest den Entspannungsdampf aus der ersten Entspannungsstufe des Entspannungskühlers zumischt. Der apparative Aufwand ist vergleichsweise gering, wenn der Mischkondensator einstufig und der Entspannungskühler zweistufig ausgebildet sind. Auch der Entspannungsdampf der zweiten Stufe des Entspannungskühlers kann für Prozesszwecke rückgewonnen werden.

Es wird ausgenutzt, dass die Verkleisterungstemperatur, bei der die Verflüssigung der Stärke des Rohstoffs einsetzt, in der Größenordnung von 90°C liegt, während für die nachfolgende Verzuckerung lediglich etwa 60°C erforderlich sind, bzw. die Fermentationstemperatur der nachfolgenden Vermaischung nur bei 35 bis 40°C liegt. Während der für die enzymatische Verflüssigung einzuhaltenden Verweilzeit, die zwischen einigen Minuten und etwa einer Stunde liegen kann, können Wärmeverluste durch geeignete Isoliermaßnahmen verhindert werden. Von besonderem Vorteil der Nutzung eines durch Entspannungsdämpfe geheizten Mischkondensators ist, dass keine Wärmeübertragungsflächen vorgesehen sind, die bei herkömmlichen Verflüssigungsverfahren zu Verkrustungen neigen.

Bei der Fermentation entsteht neben Alkohol auch Kohlendioxid (CO₂), das teilweise in der Maische gelöst ist. Das in der Maische gelöste CO₂ behindert jedoch die Funktion der Destillationskolonnen der Destillierstation und erfordert zum Teil aufwändige Konstruktionen, insbesondere da die vergorene Maische schäumt, wenn das darin gelöste CO₂ entweicht. Nicht zuletzt erhöht das in der Destillationskolonne entweichende CO₂ den Druckverlust in der Destillationskolonne.

Der aparative Aufwand der Destillationsstation kann verringert werden, wenn zwischen der Fermentierungsstation und der Destillierstation eine Entgasungsstation angeordnet ist, in welcher der Maische-Produktstrom ein aufrecht stehendes, an seinem unteren Ende evakuiertes Rohrbündel von oben nach unten durchläuft und sich in dem Rohrbündel entspannt. Der entstehende Dampf zerreibt den Maischeschaum in dem Rohrbündel, so dass die Destillationskolonnen einfacher gebaut werden können. Zudem vereinfacht sich das Vakuumsystem der Destillationskolonne. Der Entgasungsprozess wird verstärkt, wenn der Maische-Produktstrom vor dem Eintritt in das Rohrbündel einen die Maische vorerwärmenden Wärmeübertrager durchläuft. Zudem wird die Maische bereits hier näher an die Destillationstemperatur herangebracht.

Vorangegangen wurde erläutert, wie durch Abtrennung der Schalenanteile bei Körnerfrüchten als Rohstoffmaterial der Alkoholhefstellung der Trockner verkleinert werden kann. Unter Umständen führt diese Verkleinerung des Trockners dazu, dass er nicht ohne weiteres ausreichende Abdampfenergien für die Beheizung der Destillationskolonne erzeugen kann. Nachfolgend wird ein Weg erläutert, weitere Wärmeenergie für die Beheizung der Destillationskolonne aus ohnehin aufzuwendender Prozesswärme für diesen Zweck zurückgewonnen werden kann.

Es hat sich herausgestellt, dass die Restenergie des in einer Dehydrierstation entwässerten Alkohols ausreicht, um ein eventuelles Energiedefizit der Destillationsstation auszugleichen. Unter einem fünften Aspekt der Erfindung ist vorgesehen, dass der Destillationsstation eine erste, insbesondere durch Abdampf der Trockenstation beheizte Destillationskolonne aufweist, an die eine deren Rohalkohol-Produktstrom entwässernde Dehydrierstation angeschlossen ist und dass in einem Zwischenniveau der ersten Destillationskolonne oberhalb ihres Maische-Zuführniveaus eine zweite Destillationskolonne angeschlossen ist, die über einen Wärmeübertrager mit Wärme des entwässerten Alkoholdampfs der Dehydrierstation beheizt ist.

Bei einer derartigen "Side Stripper"-Anordnung wird in der zweiten Destillationskolonne Alkohol nicht mehr aus Maische abdestilliert, sondern aus einem Alkohol-Wasser-Substrat bei dementsprechend verringerter Destillationsenergie abgetrennt.

In einer bevorzugten Ausgestaltung ist der der zweiten Destillationskolonne zugeordnete Wärmeübertrager als mit entwässertem Alkoholdampf der Dehydrierstation beheizter Fallstromverdampfer ausgebildet, dem Lutterwasser aus dem Sumpf der zweiten Destillationskolonne zur Entwässerung im Kreislauf zugeführt wird. Das aus der zweiten Destillationskolonne in die ersten Destillationskolonne zurückgeführte Alkohol-Wasser-Substrat ist dementsprechend alkoholreicher als das aus der ersten Destillationskolonne der zweiten Kolonne zugeführte Substrat.

Bei der Dehydrierstation handelt es sich bevorzugt um ein Molekularsieb, dem der Rohalkohol gegebenenfalls nach einer Vorerwärmung unter einem höheren Druck als üblich, insbesondere bei einem Druck von mehr als 1,7 bar, a zugeführt wird, wird das Molekularsieb bei einem solchermaßen erhöhten Druck betrieben, lassen sich Abwärmetemperaturen des entwässerten Alkoholdampfs von mehr als 90° C erreichen und wie vorangegangen erläutert, zur Beheizung des vorzugsweise als Fallstromverdampfer ausgebildeten Wärmeübertragers nutzen.

Die Idee, die Restwärme des entwässerten Alkohol-Produktstroms in der Prozessführung an anderer Stelle einzusetzen, lässt sich auch anderweitig realisieren, beispielsweise zur Verbesserung des Wirkungsgrads der Schlempetrocknung. So kann vorgesehen sein, dass die Trocknungsstation einen die Schlempe in einen Schlempe-Dünnsaft-Produktstrom und einen Schlempe-Feststoff-Produktstrom auftrennenden Separator, beispielsweise einen Decanter oder dergleichen, sowie einen den Schlempe-Dünnsaft-Produktstrom zu Schlempe-Dicksaft eindampfenden Verdampfer umfasst. Die Trockenstation umfasst ferner einen den Schlempe-Dicksaft zusammen mit dem Schlempe-Feststoff zu Trockenschlempe fertig trocknenden Trockner. Ist auch hier der Destillierstation eine deren Rohalkohol-Produktstrom entwässernde Dehydrierstation nachgeordnet, so kann wenigstens eine Verdampferstufe des den Schlempe-Dünnsaft-Produktstrom eindampfenden Verdampfers mit Wärme des in der Dehydrierstation entwässerten Alkoholdampfs beheizt werden.

Beispielsweise kann der Verdampfer zweistufig aufgebaut sein und einen Vorverdampfer sowie einen Endverdampfer umfassen, wobei dann der Endverdampfer mit entwässertem Alkoholdampf aus der Dehydrierstation beheizt wird. Der Vorverdampfer wird zweckmäßigerweise so bemessen, dass er den zulaufenden Schlempe-Dünnsaft auf eine Feststoffkonzentration von 1 7 % bis 24 % vorkonzentriert, während der Endverdampfer dieses Vorkonzentrat zu Dicksaft (Sirup) mit einem Feststoffanteil von beispielsweise 25 bis 50 % je nach Rohstoff und zugesetzten Enzymen eindickt.

Auch unter dem letztgenannten Aspekt ist die Dehydrierstation bevorzugt als Molekularsieb ausgebildet, dem der entwässerte und gegebenenfalls auf mehr als 90°C vorerwärmte Rohalkohol bei einem Druck von mehr als 1,7 bar, a zugeführt wird.

Im Folgenden wird die Erfindung anhand einer Zeichnung näher erläutert. Hierbei zeigt:
- Figur 1: eine schematische Blockdarstellung einer erfindungsgemäßen Anlage zur Herstellung von Alkohol aus pflanzlichen Rohstoffen;
- Figur 2: eine schematische Blockdarstellung einer Verflüssigerstation der Anlage;
- Figur 3: eine schematische Blockdarstellung einer Entgasungsstation der Anlage;
- Figur 4: eine schematische Blockdarstellung einer Destillierstation mit nachfolgender Dehydrierstation der Anlage und
- Figur 5: eine schematische Blockdarstellung eines in der Anlage nach Figur 1 zur Schlempetrocknung vorgesehenen Endverdampfers.

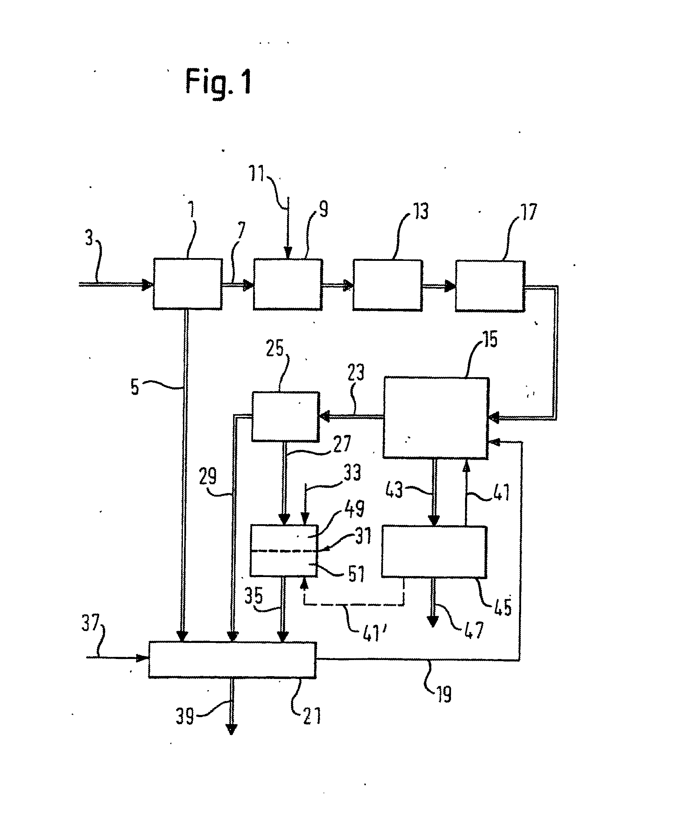

Die in Figur 1 dargestellte Anlage erlaubt es, entwässerten Ethylalkohol aus pflanzlichen, Kohlehydrate oder/und Zucker enthaltenden Rohstoffen, hier Körnerfrüchten, wie zum Beispiel Getreide oder Mais großtechnisch mit gegenüber herkömmlichen Anlagen deutlich verringertem Energieeinsatz herzustellen. Die Anlage umfasst eine Mahlstation 1, die die bei 3 als Rohstoff zugeführten Körnerfrüchte unter Abtrennung zumindest eines Teils der den Stärke oder/und Zucker enthaltenden Kern umschließenden Schalenanteile zu Mehl mit einer mittleren Korngröße von mehr als 0,2 mm, zweckmäßigerweise 0,5 bis 1 mm, vermahlt. Die Mahlstation 1 ist bevorzugt als Walzenmühle oder als Prallstrahlmühle ausgebildet. Beispiele solcher Mühlen sind zum Beispiel in "Ullmanns Encyclopädie der technischen Chemie", dritte Auflage, 1951 Verlag Urban und Schwarzenberg, Band 1, Seite 622, 623, 633, 634, beschrieben. Zweckmäßigerweise ist die Mahlstation 1 so eingestellt, dass sie die Schalenanteile in einem Gewichtsverhältnis von Schalenanteilen zu Mehlanteil von 1:9 bis 2:8 bei 5 für die nachfolgend noch näher erläuterte Weiternutzung abgibt.

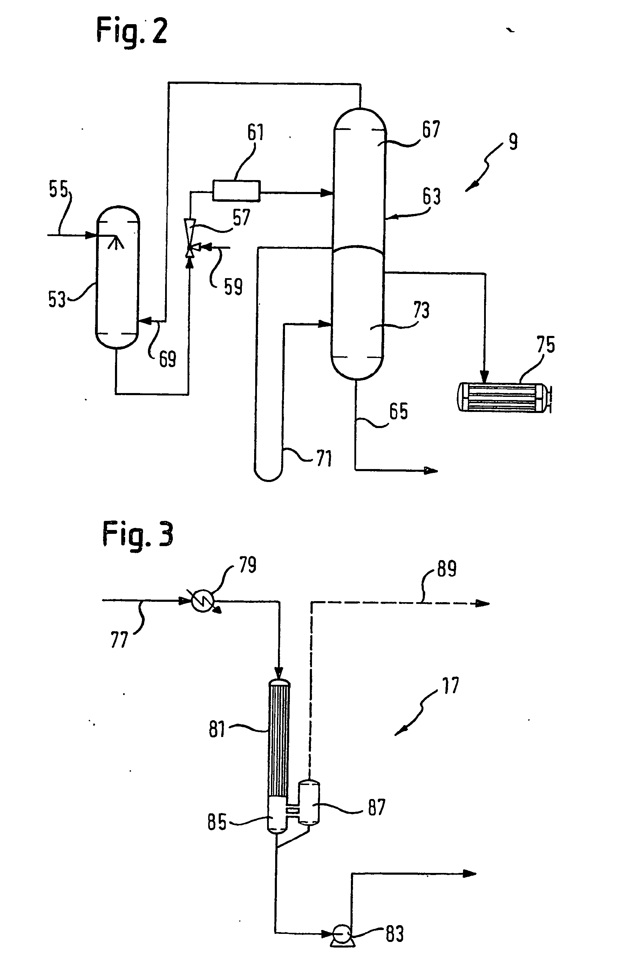

Das bei 7 abgegebene Mehl wird, gegebenenfalls nach Aufschlämmung einer Verflüssigungsstation 9 zugeführt, in welcher die zumeist kornförmige Stärke des Mehls unter Zusatz von Enzymen verflüssigt und damit in eine vergärbare Form aufgeschlossen wird. Die Stärke wird hierbei unter Zufuhr von Dampf 11 zunächst über die rohstoffabhängige Verkleisterungstemperatur erwärmt und dann wieder abgekühlt. Die Verkleisterungstemperaturen liegen für Gerste, Weizen oder Roggen in der Größenordnung von 80°C, für Mais etwa bei 75°C und für Hafer bei 85°C. Einzelheiten der Verflüssigungsstation 9 werden nachfolgend anhand von Figur 2 erläutert.

Die in der Verflüssigungsstation 9 entstehende Süßmaische wird nach Abkühlung auf Verzuckerungstemperatur von etwa 60°C unter Zugabe von Enzymen während einer Verweilzeit von 0,2 bis 5 Stunden verzuckert und nachfolgend nach weiterer Abkühlung auf Fermentationstemperatur von ca. 35 bis 40°C unter Zugabe von Hefe und gegebenenfalls weiteren Enzymen zu Alkohol vergoren. Der Verzuckerungsschritt kann gegebenenfalls entfallen, wobei dann die Süßmaische in der Verflüssigungsstation 9 unmittelbar auf die Fermentationstemperatur gekühlt wird.

In der Fermentierungsstation 13 entsteht bei der Vergärung neben Alkohol auch Kohlendioxid (CO₂), welches sich zum Teil in der bei der Fermentation entstehenden Maische löst. Das CO₂ behindert die Funktion von Destillationskolonnen einer Destillierstation 15, in welcher der in der Maische durch Vergären entstandene Alkohol abgetrennt wird. Die Maische wird deshalb der Destillierstation 15 über eine Entgasungsstation 17 zugeführt, in welcher das in der Maische gelöste CO₂ abgetrennt und zudem die Maische entschäumt wird. Einzelheiten der Entgasungsstation werden nachfolgend anhand von Figur 3 erläutert.

In der Destillierstation 15 wird die Maische erwärmt, wobei, wie nachfolgend noch näher erläutert wird, der größte Teil der hierfür erforderlichen Energie aus der Abwärme anderer Prozessstufen gewonnen wird, insbesondere dem Abdampf 19 eines Trockners 21, der die als Rückstand des Destillationsprozesses bei 23 anfallende Schlempe zu einem trockenen, auf Grund seines hohen Proteingehalts als Tierfutter verwendbaren Endprodukt (DDGS) fertig trocknet. Die bei 23 anfallende Schlempe wird zunächst in einem Separator 25, beispielsweise einem Decanter in einen Dünnsaft-Produktstrom 27 und einen Feststoff-Produktstrom (wet cake) 29 aufgeteilt. Der Dünnsaft-Produktstrom 27 wird in einem Verdampfer 31 unter Zufuhr von Energie 33 zunächst zu einem Dicksaft-Produktstrom 35 eingedampft und dann werden der Dicksaft-Produktstrom 35 und der Feststoff-Produktstrom 29 zusammen mit dem in der Mühlenstation 1 abgetrennten Schalenanteil-Produktstrom 5 dem durch Fremdenergie 37 beheizten Trockner 21 für die Endtrocknung zugeführt. Die hierbei entstehende Trockenschlempe fällt bei 39 an. Einzelheiten der Erzeugung von Trockenschlempe sind beispielsweise in der Zeitschrift "Die Branntweinwirtschaft" Mai 1976, Seiten 138 bis 141 beschrieben.

Der in dem Trockner erzeugte Abdampf hat eine Taupunkttemperatur von mehr als 95°C, bevorzugt 100 bis 105°C, arbeitet also im Wesentlichen luftfrei. Geeignet sind insbesondere Heißdampftrockner oder Rieseltrockner, wie sie beispielsweise in der genannten Literaturstelle "Ullmann's Encyclopädie der technischen Chemie", Seiten 577 und 601 beschrieben sind. Der Trockner 21 erzeugt auf diese Weise Abdampf mit einer die Destillation des Alkohols in der Destillierstation 15 erlaubenden Temperatur.
Der in der Mahlstation 1 abgetrennte Schalenanteil (Kleie) wird dem Trockner 21 direkt zugeführt, wird also nicht der Verflüssigung unterworfen und muss nicht nach dem Einmaischen wieder getrocknet werden. Der Schalenanteil des Rohstoffs dient vielmehr in dem Trockner 21 als Trägermaterial für den Feststoff-Produktstrom 29 sowie dem Dicksaft-Produktstrom 35. Auf diese Weise kann der Anteil an Trockenschlempe, der bei herkömmlichen Trocknungsverfahren als Trägermaterial für den Trocknungsprozess zurückgeführt wird, vermindert werden. Der Trockner 21 kann damit kleiner dimensioniert sein, und es verringert sich der Energiebedarf bzw. Dampfverbrauch des Trockners 21. Die Abtrennung der Schalenanteile bei 5 in der Mahlstation 1 erhöht darüber hinaus den Stärkegehalt in dem tatsächlich der Verflüssigung zugeführten Mehl, was den bei der Fermentation in der Fermentierungsstufe 13 erzielbaren Alkoholgehalt der Maische erhöht und damit den Energieverbrauch bei der Destillation der Maische vermindert.

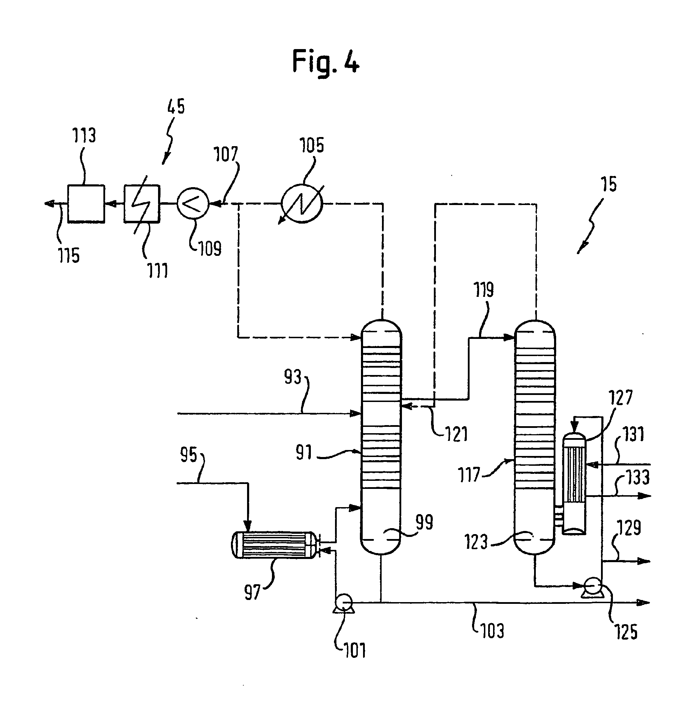

Die vorstehend erläuterte Verkleinerung des Trockners 21 kann im Einzelfall dazu führen, dass seine für den Destillationsprozess der Destillierstufe 15 ausgenutzte Abdampfwärme nicht ausreicht, um den gesamten Destillierprozess durchführen zu können. Wie im Einzelnen anhand der Figur 4 noch erläutert wird, kann zur Deckung eines eventuellen Energiedefizits die Abwärme einer den Rohalkohol der Destillierstation 15 bei 43 aufnehmenden Dehydrierstufe 45 gedeckt werden. Die Dehydrierstufe 45 ist bevorzugt als Molekularsieb ausgebildet und gibt den dehydrierten Ethylalkohol als Endprodukt bei 47 ab. Das Molekularsieb wird hierbei bei einem Druck von wenigstens 1,7 bar,a betrieben, um eine im Destillationsprozess verwertbare Abwärmetemperatur von mehr als 90°C zu erreichen.

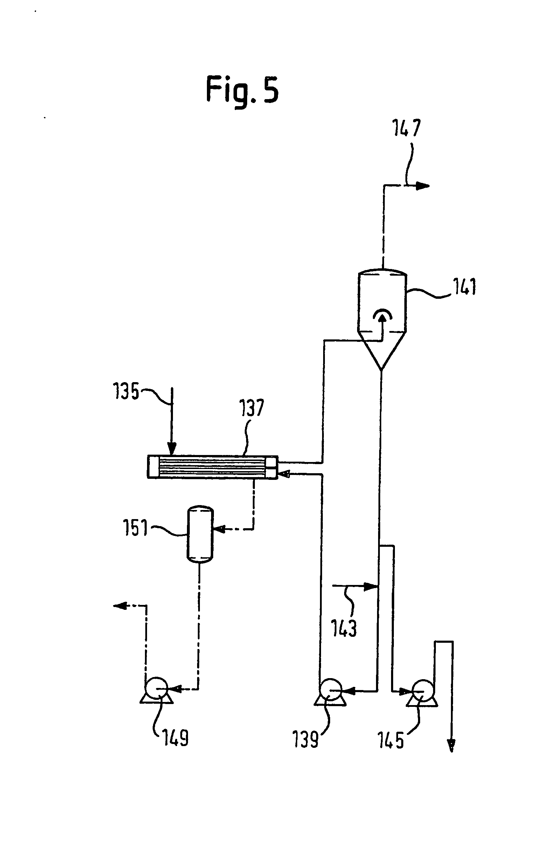

Für den Fall, dass die Prozesswärme der Destillierstufe 15 bereits allein durch den Trockner 21 gedeckt werden kann, kann die Abwärme der Dehydrierstation 45 anderweitig in dem Alkoholherstellungsprozess genutzt werden. Eine vorteilhafte Nutzung ist in dem Verdampfer 31 bei der Schlempetrocknung möglich. Der Verdampfer 31 umfasst hierzu einen beispielsweise mit Frischdampf, vorzugsweise aber mit mechanischer Brüdenverdichtung betriebenen Vorverdampfer 49, der den Feststoffanteil in dem bei 27 zulaufenden Schlempe-Dünnsaft auf 17 % bis 24 % vorkonzentriert, während ein im Produktstrom nachgeschalteter Endverdampfer 51 für eine weitergehende Konzentration des Feststoffanteils auf 25 % bis 50 % je nach Rohstoff oder zugesetzten Enzymen sorgt. Soweit die Abwärmeenergie der Dehydrierstation 45 nicht in der Destillierstation 15 erforderlich ist, kann sie, wie bei 41' dargestellt, auch zur Erwärmung des Endverdampfers 51 genutzt werden. Damit muss lediglich der Vorverdampfer 49 durch die bei 33 dem Verdampfer 31 zugeführte, bei mechanischer Brüdenverdichtung elektrische Energie erwärmt werden. Einzelheiten des Endverdampfers 51 werden nachfolgend anhand von Figur 5 erläutert.

Figur 2 zeigt Einzelheiten der Verflüssigungsstation 9. Diese umfasst einen Mischkondensator 53, der das bei 55 aus der Mahlstation 1. in gegebenenfalls aufgeschlämmter Form und mit für die Verflüssigung erforderlichen Enzymen versetzte Mehl auf eine Temperatur unterhalb der Verkleisterungstemperatur des für die Alkoholherstellung benutzten Rohstoffs erwärmt. Das solchermaßen vorerwärmte Produkt wird in einem Dampfstrahlinjektor 57 mit Frischdampf 59 vermischt und dadurch auf eine Temperatur über der Verkleisterungstemperatur des Rohstoffs erwärmt und einer Verweilstrecke 61 zugeführt, in der es eine gewisse Zeit von zum Beispiel 0,1 bis 1 Stunde oberhalb der Verkleisterungs- oder Verflüssigungstemperatur gehalten wird. Danach wird das verflüssigte Produkt in einem mehrstufigen Entspannungskühler 63 auf die für die Weiterverarbeitung der am Ausgang 65 anfallenden Süßmaische erforderliche Temperatur gekühlt. Der in der ersten Stufe 67 des Entspannungskühlers 63 anfallende Entspannungsdampf wird bei 69 dem Mischkondensator 53 unmittelbar zugeführt. Damit ist eine Wärmerückgewinnung möglich, ohne dass zur Verkrustung neigende Wärmeübertragungsflächen benutzt werden müssen. Die über einen Druckausgleichsiphon 71 an die erste Entspannungsstufe 67 des Entspannungskühlers 63 angeschlossene, die Süßmaische bei 65 abgebende zweite Entspannungsstufe 73 des Entspannungskühlers 63 führt ihren Entspannungsdampf einem Wärmeübertrager 75 für eine anderweitige Rückgewinnung der Wärme zu.

Der Mischkondensator 53 erwärmt das Produkt bis auf wenige Grad Celsius unterhalb der Verkleisterungstemperatur, so dass der Dampfstrahlinjektor mit vergleichsweise geringem Dampfverbrauch die Produkttemperatur auf wenige Grad oberhalb der Verkleisterungstemperatur erhöhen kann. Der Energiebedarf des Mischkondensators 53 kann hierbei vollständig durch den Entspannungsdampf des Entspannungskühlers 9 gedeckt werden.

Im vorliegenden Ausführungsbeispiel ist lediglich der Entspannungskühler 9 mehrstufig ausgebildet. Es versteht sich, dass auch der Mischkondensator 53 mehrere Stufen haben kann, die dann jeweils gesondert durch einzeln zugeordnete Stufen des Entspannungskühlers mit Entspannungsdampf versorgt werden.

Figur 3 zeigt Einzelheiten der Entgasungsstation 17. Die bei 77 aus der Fermentierungsstation 13 zugeführte, alkoholhaltige Maische wird zunächst in einem Wärmeübertrager 79 vorerwärmt und dann am oberen Ende eines mit seinem Rohrbündel aufrecht stehenden Rohrbündel-Entspanners 81 versprüht, so dass sie sich in den Rohren entspannen kann, bevor die entgaste Maische mittels einer Austragpumpe 83 aus dem an das untere Ende des Rohrbündels anschließenden Sumpf abgezogen und der Destillierstation 15 zugeführt wird. Der Sumpf 85 bildet einen ersten Abscheider, der mit einem seitlich angeordneten Flüssigkeitsabscheider 87 kommuniziert. Der Flüssigkeitsabscheider 87 ist bei 89 an ein Vakuumsystem angeschlossen, welches CO₂ sowie Wasserdämpfe und Alkoholdämpfe abzieht. Die Anordnung ist so getroffen, dass am unteren Ende des Rohrbündels ein Unterdruck entsteht und die Maische mit einer Geschwindigkeit von etwa 20 bis 60 m/s durch das Rohrbündel strömt. Dies fördert die Entgasung der Maische und sorgt insbesondere dafür, dass der bei der Entspannung entstehende Dampf den Schaum der Maische zerreibt. Da die Maische entgast ist, kann die nachfolgend erläuterte Destillationskolonne der Destillierstation 15 einfacher gebaut werden und auch das Vakuumsystem der Destillationskolonne vereinfacht sich. Damit einher geht auch eine Verringerung des Druckverlustes in der Destillationskolonne.

Einzelheiten der Destillierstation 15 sind in Figur 4 dargestellt. Die Destillierstation 15 umfasst eine erste Rektifizier- oder Destillationskolonne 91, der in einem Zwischenniveau bei 93 die entgaste Maische aus der Entgasungsstation 17 zugeführt wird. Der bei 95 zugeführte Abdampf des Trockners 21 speist einen Wärmeübertrager 97, durch den die im Sumpf 99 der Destillationskolonne 91 sich sammelnde Schlempe mittels einer Zirkulationspumpe 101 im Kreislauf geführt und erwärmt wird. Aus dem Sumpf 99 wird bei 103 darüber hinaus die dem Separator 25 für den Trocknungsvorgang zugeführte Schlempe abgezogen. Im oberen Bereich der Destillationskolonne 91 wird in üblicher Weise Alkohol und Wasser enthaltendes Dampf-Flüssigkeits-Gemisch über einen Kondensator 105 im Kreislauf geführt und bei 107 als wasserhaltiger Rohalkohol mittels einer Pumpe 109 abgezogen. Der abgezogene Rohalkohol wird in einem Verdampfer 111 auf eine Temperatur von mehr als 90°C erwärmt und unter dem Druck der Pumpe 109 einem die Dehydrierstation 45 bildenden Molekularsieb 113 zugeführt. Das Molekularsieb 113 entwässert den Rohalkohol und liefert bei 115 als Endprodukt der Alkoholherstellung wasserfreien Ethylalkohol. Wesentlich ist, dass die Pumpe 109 den Druck in dem Molekularsieb 113 auf mehr als 1,7 bar, a erhöht, um auf diese Weise den bei 115 verfügbaren Produktstrom aus dehydriertem Alkoholdampf für die Abwärmerückgewinnung in nachfolgend noch näher erläuterter Weise nutzen zu können.

Während die Destillationskolonne 91 ausschließlich von der Abwärme des Trockners 21 mit Prozesswärme versorgt wird, wird eine zweite Destillationskolonne 117, die nach Art eines "side strippers" mit der Destillationskolonne 91 verbunden ist, im Wesentlichen ausschließlich durch die Abwärme des aus dem Molekularsieb 113 austretenden entwässerten Alkoholdampfs mit Prozesswärme versorgt. Die Destillationskolonne 117 entnimmt an einem Niveau oberhalb des Maische-Zuführungsniveaus 93 der Destillationskolonne 91 ein Alkohol/Wasser-Gemisch, welches bei 119 dem Kopf der Destillationskolonne 117 zugeführt wird und nach Alkoholanreicherung an einem etwas tiefer gelegenen Niveau bei 121 in die Destillationskolonne 91 zurückgeführt wird. Der im Wesentlichen maischefreie Sumpf 123 der Destillationskolonne 117 enthält Lutterwasser, das mittels einer Zirkulationspumpe 125 im Kreislauf durch einen Fallstromverdampfer 127 geführt wird, so dass bei 129 Wasser aus der Destillationskolonne 117 abgezogen werden kann. Der Fallstromverdampfer 127 wird durch den auf wenigstens 90°C erwärmten wasserfreien Alkoholdampf aus dem Molekularsieb 113 beheizt, wobei der wasserfreie Alkoholdampf bei 131 zugeführt und bei 133 als gekühltes Endprodukt der Alkoholherstellungsanlage abgeführt wird. Beispiele geeigneter Fallstromverdampfer sind beispielsweise in CH 510 450 oder DE 1 519 714 A beschrieben. Ein Beispiel eines Molekularsiebs ist in US 4 407 662 beschrieben.

Die vorstehend erläuterte Destillierstation 15 kann auf Grund der Wärmerückgewinnung aus dem Produktstrom des entwässerten Ethylalkohols auch dann betrieben werden, wenn der Trockner 21 bei Verkleinerung der Trocknerkapazität wegen der Zuführung des in der Mahlstation abgetrennten Kleie- oder Schalenanteils des Rohstoffs nicht hinreichend Energie für das Betreiben des gesamten Destillationsprozesses liefert. Reicht die Abwärme des Trockners 21 jedoch aus, so kann die Abwärme des Molekularsiebs 113 auch anderweitig rückgewonnen werden, insbesondere als Prozesswärme im Endverdampfer 51.

Wie Figur 5 zeigt, wird der im Molekularsieb 113 bei einer Temperatur von mehr als 90°C und einem Druck von mehr als 1,7 bar,a entwässerte Ethanoldampf bei 135 einem als Zwangsumlauf-Verdampferheizkörper ausgebildeten Wärmeübertrager 137 zugeführt und erwärmt dort den von einer Zirkulationspumpe 139 im Kreislauf durch den Wärmeübertrager 137 und einen Entspannungsbehälter 141 geförderten, bei 143 dem Eindampfungskreislauf zugeführten, im Vorverdampfer 49 vorkonzentrierten Schlempesaft. Eine Austragpumpe 145 entnimmt an einer im Kreislauf vor dem Saftzulauf 143 gelegenen Stelle den Dickschlempesaft und führt ihn dem Trockner 21 zu. Im Entspannungsbehälter 141 entstehender Wasserdampf wird bei 147 einem nicht näher dargestellten Kondensationssystem oder weiteren Verdampfer zugeführt. Der in dem Wärmeübertrager 137 abgekühlte, entwässerte Alkoholdampf wird mittels einer Austragpumpe 149, die gegebenenfalls über einen Ausgleichs- oder Vorlagebehälter 151 an dem Wärmeübertrager 137 angeschlossen ist, als Endprodukt der Alkoholherstellung abgeführt.

## Patentansprüche

1. Anlage zur Herstellung von Alkohol aus Körnerfrucht-Rohstoffen, umfassend
- eine zumindest den die Stärke oder/und Zucker enthaltenden Kern der Körnerfrüchte zu Mehl vermahlende Mahlstation (1),
- eine das Mehl zu verflüssigtem Rohstoff aufschließende Verflüssigungsstation (9),
- eine verflüssigten Rohstoff zu Maische vergärende Fermentierungsstation (13),
- eine den Alkohol aus der Maische abtrennende Destillierstation (15) und
- eine Trocknungsstation (21, 31) für in der Destillierstation (15) anfallende Schlempe,
**dadurch gekennzeichnet, dass**
die Mahlstation (1) die Körnerfrüchte unter Abtrennung zumindest eines Teils' ihrer den Kern umschließenden Schalenanteile zu Mehl vermahlt und die abgetrennten Schalenanteile der Trockenstation (21, 31) als Trägermedium für die Schlempetrocknung zugeführt sind,
dass die Trocknungsstation (21, 31) einen die in der Destillierstation (15) anfallende Schlempe endtrocknenden Trockner (21) mit einer Taupunkttemperatur von mehr als 95°C, bevorzugt 100° bis 105°C umfasst und dass die Destillierstation (15) eine mit dem Abdampf des Trockners (21) beheizte Destillationskolonne (91) umfasst.

2. Anlage nach Anspruch 1, **dadurch gekennzeichnet, dass** die Mahlstation (1) die Schalenanteile in einem Gewichtsverhältnis Schalenanteile zu Mehl von 1 zu 9 bis 2 zu 8 abtrennt.

3. Anlage nach Anspruch 1 oder 2, **dadurch gekennzeichnet, dass** die Mahlstation (1) die Körnerfrüche zu Mehl mit einer mittleren Korngröße zwischen 0,5 und 1 mm vermahlt.

4. Anlage nach einem der Ansprüche 1 bis 3, **dadurch gekennzeichnet, dass** die Mahlstation (1) eine Walzenmühle oder eine Prallstrahlmühle aufweist.

5. Anlage nach einem der Ansprüche 1 bis 4, **dadurch gekennzeichnet, dass** zur Herstellung von Alkohol aus pflanzlichen Rohstoffen, insbesondere zu Mehl vermahlenen Körnerfrüchten, eine Kohlehydrate und/oder Zucker in dem Rohstoff aufschließende Verflüssigungsstation (9) mit einem dem Produktstrom des zerkleinerten Rohstoffs oder einer Aufschlämmung hiervon Dampf zumischenden, wenigstens eine Mischstufe umfassenden Mischkondensator (53), einen dem Mischkondensator (53) nachgeschalteten, dem Produktstrom Heißdampf zumischenden Dampfstrahlinjektor (57) und einem dem Dampfstrahlinjektor (57) nachgeschalteten, wenigstens eine Entspannungsstufe (67, 73) umfassenden Entspannungskühler (63) für den Produktstrom vorgesehen ist,
wobei der Mischkondensator (53) den Produktstrom Entspannungsdampf des Entspannungskühlers (63) zumischt.

6. Anlage nach Anspruch 5, **dadurch gekennzeichnet, dass** zumindest der Entspannungskühler (63) mehrstufig ist und der Mischkondensator (53) den Produktstrom zumindest den Entspannungsdampf der ersten Entspannungsstufe (67) des Entspanungskühlers (67) zumischt.

7. Anlage nach Anspruch 6, **dadurch gekennzeichnet, dass** der Mischkondensator (53) einstufig und der Entspannungskühler (63) zweistufig ausgebildet ist.

8. Anlage nach einem der Ansprüche 5 bis 7, **dadurch gekennzeichnet, dass** der Mischkondensator (53) den Produktstrom auf eine Temperatur unterhalb der Verkleisterungstemperatur des Rohstoffs und der Dampfstrahlinjektor (57) den Produktstrom auf eine Temperatur oberhalb der Verkleisterungstemperatur des Rohstoffs erwärmt.

9. Anlage nach einem der Ansprüche 1 bis 8, **dadurch gekennzeichnet, dass** zwischen der Fermentierungsstation (13) und der Destillierstation (15) eine Entgasungsstation (17) angeordnet ist, in welcher der Maische-Produktstrom ein aufrecht stehendes, an seinem unteren Ende evakuiertes Rohrbündel (81) von oben nach unten durchläuft und sich in dem Rohrbündel (81) entspannt.

10. Anlage nach Anspruch 9, **dadurch gekennzeichnet, dass** der Maische-Produktstrom vor dem Eintritt in das Rohrbündel (81) einen die Maische vorerwärmenden Wärmeübertrager (79) durchläuft.

11. Anlage nach einem der Ansprüche 1 bis 10, **dadurch gekennzeichnet, dass** der Trockner (21) im Wesentlichen luftfreien Abdampf erzeugt.

12. Anlage nach einem der Ansprüche 1 bis 11, **dadurch gekennzeichnet, dass** der Trockner (21) als Heißdampftrockner ausgebildet ist.

13. Anlage nach einem der Ansprüche 1 bis 12, **dadurch gekennzeichnet, dass** die Destillierstation (15) eine erste, insbesondere durch Abdampf der Trockenstation (21, 31) beheizte Destillationskolonne (91) aufweist, an die eine deren Rohalkohol-Produktstrom entwässernde Dehydrierstation (45) angeschlossen ist
und dass in einem Zwischenniveau der ersten Destillationskolonne (91) oberhalb ihres Maische-Zuführniveaus (93) eine zweite Destillationskolonne (117) angeschlossen ist, die über einen Wärmeübertrager (127) mit Wärme des entwässerten Alkoholdampfs der Dehydrierstation (45) beheizt ist.

14. Anlage nach Anspruch 13, **dadurch gekennzeichnet, dass** der Wärmeübertrager als mit entwässertem Alkoholdampf der Dehydrierstation (45) beheizten Fallstromverdampfer (127) ausgebildet ist.

15. Anlage nach Anspruch 13 oder 14, **dadurch gekennzeichnet, dass** die Dehydrierstation (45) ein Molekularsieb (117) umfasst.

16. Anlage nach Anspruch 15, **dadurch gekennzeichnet, dass** das Molekularsieb (117) bei einem Druck von 1,7 bar,a oder mehr betrieben ist.

17. Anlage nach einem der Ansprüche 1 bis 16, **dadurch gekennzeichnet, dass** die Trocknungsstation (21, 31) einen die Schlempe in einen Schlempe-Dünnsaft-Produktstrom und einen Schlempe-Feststoff-Produktstrom trennenden Separator (25), einen den Schlempe-Dünnsaft-Produktstrom zu Schlempe-Dicksaft eindampfenden Verdampfer (31) sowie einen den Schlempe-Dicksaft zusammen mit dem Schlempe-Feststoff zu Troskenschlempe trocknenden Trockner (21) umfasst,
dass der Destillierstation (15) eine den Rohalkohol-Produktstrom entwässernde Dehydrierstation (45) nachgeordnet ist
und dass der Verdampfer (31) wenigstens eine mit Wärme des entwässerten Alkoholdampfs der Dehydrierstation beheizte. Verdampferstufe (51) aufweist.

18. Anlage nach Anspruch 17, **dadurch gekennzeichnet, dass** der Verdampfer (31) einen Vorverdampfer (49) und einen Endverdampfer (51) umfasst und dass der Endverdampfer (51) mit entwässertem Alkoholdampf aus der Dehydrierstation (45) beheizt ist.

19. Anlage nach Anspruch 17 oder 18, **dadurch gekennzeichnet, dass** die Dehydrierstation (45) ein Molekularsieb (117) umfasst.

20. Anlage nach Anspruch 19, **dadurch gekennzeichnet, dass** das Molekularsieb (117) bei einem Druck von 1,7 bar, a oder mehr betrieben ist.

## Claims

1. Apparatus for producing alcohol from cereal raw materials, comprising
- a grinding station (1) which grinds to flour at least the grain comprising the starch and/or sugar of the cereals,
- a liquefaction station (9) which digests the flour to liquefied raw material,
- a fermentation station (13) which ferments liquefied raw material to mash,
- a distillation station (15) separating off the alcohol from the mash and
- a drying station (21, 31) for vinasse arising in the distillation station (15),
**characterized in that**
the grinding station (1) grinds to flour the cereals with removal of at least part of the seed coat portions surrounding the grain and the removed seed coat portions are fed to the drying station (21, 31) as carrier medium for the vinasse drying, **in that** the drying station (21, 31) comprises a drier (21) which carries out the final drying of the vinasse produced as a residue in the distillation station (15) with a dew point temperature of above 95°C, preferably 100°C to 105°C, and **in that** the distillation station (15) comprises a distillation column (91) heated by the vapor of the drier (21).

2. Apparatus according to Claim 1, **characterized in that** the grinding station (1) separates off the seed coat portions in a weight ratio of seed coat portions to flour of 1 to 9 to 2 to 8.

3. Apparatus according to Claim 1 or 2, **characterized in that** the grinding station (1) grinds the cereals to flour with a mean particle size between 0.5 and 1 mm.

4. Apparatus according to one of Claims 1 to 3, **characterized in that** the grinding station (1) has a roller mill or an impact jet mill.

5. Apparatus according to one of Claims 1 to 4, **characterized in that**, for producing alcohol from plant raw materials, in particular cereals ground to flour, a liquefaction station (9) digesting carbohydrates and/or sugars in the raw material is provided, which has a mixing condenser (53) comprising at least one mining stage and admixing steam to the product stream of the comminuted raw material or a suspension thereof,
a steam-jet injector (57) downstream of the mixing condenser (53) and admixing superheated steam to the product stream, and an expansion cooler (63) for the product stream connected downstream of the steam-jet injector (57) and comprises at least one expansion stage (67, 73),
the mixing condenser (53) admixing expansion vapor of the expansion cooler (63) to the product stream.

6. Apparatus according to Claim 5, **characterized in that** at least the expansion cooler (63) is of multistage construction and the mixing condenser (53) admixes to the product stream at least the expansion vapor of the first expansion stage (67) of the expansion cooler (67).

7. Apparatus according to Claim 6, **characterized in that** the mixing condenser (53) is of single-stage construction, and the expansion cooler (63) is of two-stage construction.

8. Apparatus according to one of Claims 5 to 7, **characterized in that** the mixing condenser (53) heats the product stream to a temperature below the gelatinization temperature of the raw material, and the steam-jet injector (57) heats the product stream to a temperature above the gelatinization temperature of the raw material.

9. Apparatus according to one of Claims 1 to 8, **characterized in that**, between the fermentation station (13) and the distillation station (15), there is disposed a degassing station (17) in which the mash product stream passes through from top to bottom a vertically standing tube bundle (81) which is evacuated at its bottom end, and the mash product stream expands in the tube bundle (81).

10. Apparatus according to Claim 9, **characterized in that** the mash product stream, before entry into the tube bundle (81), passes through a heat exchanger (79) preheating the mash.

11. Apparatus according to one of Claims 1 to 10, **characterized in that** the drier (21) produces essentially air-free exhaust vapor.

12. Apparatus according to one of Claims 1 to 11, **characterized in that** the drier (21) is constructed as a superheated steam drier.

13. Apparatus according to one of Claims 1 to 12, **characterized in that** the distillation station (15) has a first distillation column (91) which is heated, in particular, by exhaust vapor of the drying station (21, 31), to which is connected a dehydration station (45) which dehydrates its crude alcohol product stream
and **in that** a second distillation column (117) is connected at an intermediate level of the first distillation column (91) above its mash feed level (93), which second distillation column is heated via a heat exchanger (127) by heat of the dehydrated alcohol vapor of the dehydration station (45).

14. Apparatus according to Claim 13, **characterized in that** the heat exchanger is constructed as a falling-film evaporator (127) heated by dehydrated alcohol vapor of the dehydration station (45).

15. Apparatus according to Claim 13 or 14, **characterized in that** the dehydration station (45) comprises a molecular sieve (117).

16. Apparatus according to Claim 15, **characterized in that** the molecular sieve (117) is operated at a pressure of 1.7 bar absolute or more.

17. Apparatus according to one of Claims 1 to 16, **characterized in that** the drying station (21, 31) comprises a separator (25) which separates the vinasse into a vinasse-thin juice product stream and a vinasse-solids product stream, an evaporator (31) which evaporates the vinasse-thin juice product stream to form vinasse-thick juice, and also a drier (21) which dries the vinasse-thick juice together with the vinasse-solids to give dry vinasse,
**in that** the distillation station (15) is connected downstream of the dehydration station (45) which dehydrates the crude alcohol product stream
and **in that** the evaporator (31) has at least one evaporator stage (51) heated by heat of the dehydrated alcohol vapor of the dehydration station.

18. Apparatus according to Claim 17, **characterized in that** the evaporator (31) comprises a pre-evaporator (49) and a final evaporator (51) and **in that** the final evaporator (51) is heated by dehydrated alcohol vapor from the dehydration station (45).

19. Apparatus according to Claim 17 or 18, **characterized in that** the dehydration station (45) comprises a molecular sieve (117).

20. Apparatus according to Claim 19, **characterized in that** the molecular sieve (117) is operated at a pressure of 1.7 bar absolute or more.

## Revendications

1. Installation pour fabriquer de l'alcool à partir de matières premières de grains comprenant
- une station de broyage (1) broyant au moins le noyau contenant l'amidon et/ou le sucre des grains en farine,
- une station de liquéfaction (9) désagrégeant la farine en matière première liquéfiée,
- une station de fermentation (13) fermentant de la matière première liquéfiée en moût,
- une station de distillation (15) séparant l'alcool du moût et
- une station de séchage (21, 31) pour la vinasse obtenue à la station de distillation (15),
**caractérisée en ce que**
la station de broyage (1) broie les grains en séparant au moins une partie de leurs fractions de coque entourant le noyau pour former de la farine et les fractions de coque séparées sont amenées à la station de séchage (21, 31) comme agent support pour le séchage de la vinasse,
**en ce que** la station de séchage (21, 31) comprend un sécheur (21) assurant le séchage final de la vinasse produite à la station de distillation (15) avec une température du point de rosée de plus de 95°C, de préférence 100° à 105°C et **en ce que** le station de distillation (15) comprend une colonne de distillation (91) chauffée avec la vapeur évacuée du sécheur (21).

2. Installation selon la revendication 1, **caractérisée en ce que** la station de broyage (1) sépare les fractions de coque dans un rapport de poids fractions de coque/farine de 1/9 à 2/8.

3. Installation selon la revendication 1 ou 2, **caractérisée en ce que** la station de broyage (1) broie les graines en farine avec une grosseur de grain moyenne comprise entre 0,5 et 1 mm.

4. Installation selon l'une quelconque des revendications 1 à 3, **caractérisée en ce que** la station de broyage (1) présente un broyeur à cylindres ou un moulin à jet de percussion.

5. Installation selon l'une quelconque des revendications 1 à 4, **caractérisée en ce que**, pour fabriquer de l'alcool à partir de matières premières végétales, en particulier des grains broyés en farine, il est prévu une station de liquéfaction (9) désagrégeant les hydrates de carbone et/ou du sucre dans la matière première avec un condensateur de mélange (53) ajoutant de la vapeur au flux de produit de la matière première broyée ou à une suspension de cette matière, comprenant au moins un niveau de mélange, un injecteur à jet de vapeur (57) placé en aval du condensateur de mélange (53) et ajoutant de la vapeur chaude au flux de produit et un refroidisseur de détente (63) placé en aval de l'injecteur à jet de vapeur (57) et comprenant au moins un niveau de détente (67, 73) pour le flux de produit,
le condensateur de mélange (53) ajoutant de la vapeur de détente du refroidisseur de détente (63) au flux de produit.

6. Installation selon la revendication 5, **caractérisée en ce qu'**au moins le refroidisseur de détente (63) est à plusieurs niveaux et le condensateur de mélange (53) ajoute au flux de produit au moins la vapeur de détente du premier niveau de détente (67) du refroidisseur de détente (67).

7. Installation selon la revendication 6, **caractérisée en ce que** le condensateur de mélange (53) est réalisé avec un niveau et le refroidisseur de détente (63) avec deux niveaux.

8. Installation selon l'une quelconque des revendications 5 à 7, **caractérisée en ce que** le condensateur de mélange (53) réchauffe le flux de produit à une température située au-dessous de la température de gélatinisation de la matière première et l'injecteur à jet de vapeur (57) réchauffe le flux de produit à une température située au-dessus de cette température.

9. Installation selon l'une quelconque des revendications 1 à 8, **caractérisée en ce qu'**entre la station de fermentation (13) et la station de distillation (15) est disposée une station de dégazage (17), dans laquelle le flux de produit de moût traverse un ensemble de tuyaux (81) disposés verticalement et mis sous vide sur son extrémité inférieure du haut vers le bas et se détend dans l'ensemble de tuyaux (81).

10. Installation selon la revendication 9, **caractérisée en ce que** le flux de produit de moût traverse un échangeur de chaleur (79) préchauffant le moût avant l'entrée dans l'ensemble de tuyaux (81).

11. Installation selon l'une quelconque des revendications 1 à 10, **caractérisée en ce que** le sécheur (21) génère essentiellement de la vapeur de sortie sans air.

12. Installation selon l'une quelconque des revendications 1 à 11, **caractérisée en ce que** le sécheur (21) est réalisé sous forme de sécheur de vapeur chaude.

13. Installation selon l'une quelconque des revendications 1 à 12, **caractérisée en ce que** la station de distillation (15) présente une première colonne de distillation (91) chauffée en particulier par la vapeur évacuée de la station de séchage (21, 31), colonne à laquelle est raccordée une station de déshydrogénation (45) déshydratant son flux de produit d'alcool brut
et **en ce que** dans un niveau intermédiaire de la première colonne de distillation (91) au-dessus de son niveau d'arrivée de moût (93) est raccordée une seconde station de distillation (117), qui est chauffée au moyen d'un échangeur de chaleur (127) avec de la chaleur de la vapeur d'alcool déshydratée de la station de déshydrogénation (45).

14. Installation selon la revendication 13, **caractérisée en ce que** l'échangeur de chaleur est conçue sous la forme d'un évaporateur à flot tombant (127) chauffé avec de la vapeur d'alcool déshydratée de la station de déshydrogénation (45).

15. Installation selon la revendication 13 ou 14, **caractérisée en ce que** la station de déshydrogénation (45) comprend un tamis moléculaire (117).

16. Installation selon la revendication 15, **caractérisée en ce que** le tamis moléculaire (117) est exploité à une pression de 1,7 bars, ou plus.

17. Installation selon l'une quelconque des revendications 1 à 16, **caractérisée en ce que** la station de séchage (21, 31) comprend un séparateur (25) séparant la vinasse dans un flux de produit vinasse-jus léger et un flux de produit vinasse-matière solide, un évaporateur (31) réduisant par ébullition le flux de produit vinasse-jus léger en sirop de vinasse et un sécheur (21) séchant le sirop de vinasse conjointement avec le produit solide de vinasse pour former de la vinasse sèche,
**en ce que** la station de déshydrogénation (45) déshydratant le flux de produit d'alcool brut est placée en aval de la station de distillation (15)
et **en ce que** l'évaporateur (31) présente au moins un niveau d'évaporateur (51) chauffé avec de la chaleur de la vapeur d'alcool déshydratée de la station de déshydrogénation.

18. Installation selon la revendication 17, **caractérisée en ce que** l'évaporateur (31) comprend un pré-évaporateur (49) et un évaporateur final (51) et **en ce que** l'évaporateur final (51) est chauffé avec de la vapeur d'alcool déshydratée provenant de la station de déshydrogénation (45).

19. Installation selon la revendication 17 ou 18, **caractérisée en ce que** la station de déshydratation comprend un tamis moléculaire (117).

20. Installation selon la revendication 19, **caractérisée en ce que** le tamis moléculaire (117) est exploité à une pression de 1,7 bar, ou plus.
